# EUROPEAN PATENT SPECIFICATION

(11) **EP 0 608 386 B1**
(45) Date of publication and mention of the grant of the patent: **29.05.1996**
(21) Application number: 93900025.3
(22) Date of filing: 09.10.1992
(51) Int. Cl.: C12M 1/00

(54) **A METHOD FOR CONTINUOUSLY RECOVERING MICROBIAL FERMENTATION PRODUCTS AND/OR CELLULAR SUBSTANCE**
VERFAHREN ZUR KONTINUIERLICHEN GEWINNUNG VON MIKROBIELLEN FERMENTATIONSPRODUKTEN UND/ODER ZELLSUBSTANZ
PROCEDE DE RECUPERATION EN CONTINU DE PRODUITS DE LA FERMENTATION MICROBIENNE ET/OU DE SUBSTANCES CELLULAIRES

(30) Priority: 09.10.1991 DE 4133383
(43) Date of publication of application: 03.08.1994
(73) Proprietor: KULOZIK, Ulrich, W-8050 Freising (DE)
(72) Inventor: KULOZIK, Ulrich, W-8050 Freising (DE)
(74) Representative: Thiel, Christian, Dr. Dipl.-Chem.
(86) International application number: EP9202323
(87) International publication number: WO9307253

(56) References cited:
- EP-A- 0 185 407
- DE-A- 2 812 360

## Description

The present invention relates to a method for continuously recovering microbial fermentation products and/or microbial cellular substance in a tubular fermentation reactor to which substrate and possibly microorganism are continuously fed and from which product phase is continuously removed, and to the use of an apparatus for carrying out this method. The method and apparatus are in particular suitable for recovering lactic acid and lactic-acid bacteria.

The industrial recovery of lactic acid by microbial fermentation has hitherto operated solely in the discontinuous process mode. This results in the basic disadvantage that prior art reactors can only work with an extremely low volumetric productivity at the threshold of economy.

For this reason methods have been developed for clearly increasing productivity. An essential feature of these developments, that have not yet proved their industrial feasibility, is the continuous process mode. It is characterized by a retention of the microorganism cells that, freely suspended in the medium, have optimal contact and exchange with the nutrients and are retained in the reactor by membrane filters or centrifuges, while the serum phase of the cell suspension is separated at a certain exchange rate determining the turnover time and is replaced by new fermentation substrate. Since the cells are not washed out of the reactor they can develop into substantially higher cell densities than is possible in the discontinuous process due to the necessary interruptions and constantly recurring start-up and shut-down phases of the unit.

Despite the continuous process mode a productivity lead over the conventional batch process can be achieved only at very high cell concentrations that are hardly controllable technically or physiologically. The reason for this observation readily made in experiments is that the same high final-product concentration exists in a large-volume stirrer vessel reactor with complete reaction of the carbohydrate source as in the discharge of the continuously operated reactor due to the almost ideal intermixture at every place, i.e. throughout the reaction space. The consequence is a massive feedback inhibition of the microorganisms, i.e. a dramatically reduced reaction speed which can be compensated only by an immensely high cell density. However populations with high cell density have a very high viscosity, and the problem of a vitality loss of the culture arises in the course or time.

Conversely, it is possible to reduce the high final-product concentration by increasing the flow rate, i.e. reducing the turnover time of the substrate, but the result of this is an incomplete yield.

Another adverse property of stirrer vessel reactors is that the stirring sets the total fermenter content rotating without bringing about the desired effect of good intermixture. Only flow breakers prevent this rotation but they convert the surplus energy into heat that must ultimately be removed again unused by heat exchangers.

Summing up, continuous fermentation processes in conventional stirrer vessel reactors can be characterized by saying that the technical and economic objectives "high productivity" and "complete yield" conflict with each other and can only be realized alternatively.

A continuous fermentation method in a tubular reactor is disclosed in DE-A 2 812 360, without giving details on the type of flow in that reactor.

The invention is accordingly based on the problem of providing a continuous fermentation method that avoids the disadvantages of the above methods and in particular permits virtually complete utilization of the available substrate.

This problem is solved by a method of the type described at the outset that is carried out in a suitable medium in a tubular reactor through which the medium flows turbulently in a plug flow at a Reynolds number of flow of Re ≥ 2500 width fresh substrate being fed in accordance with a turnover time specific to the organism.

The term substrate refers according to the invention to a customary nutrient medium that contains all nutrients and salts necessary for recovering the desired fermentation product or cellular substance and is set to a suitable pH value. The term medium refers to the fermentation medium comprising substrate, product and additives that is present in the reactor.

The reactor used in accordance with the invention comprises a long slender tubular path through which flow is turbulent. Stirring elements are unnecessary so that a very effective use of energy is given. This tubular reactor is operated continuously and preferably with cell retention. The fermentative reaction takes place along the tubular path, and concentration gradients develop in the direction of flow. To the degree that the carbohydrate source gradually reacts, a reaction product concentration builds up in an inverse form. High concentrations of the inhibiting final product (lactate) thus occur only in the last, relatively small zone of the tubular path, while the microorganisms in the front zone can work at maximum reaction speed almost uninhibited physiologically. Furthermore the microorganisms in the front part of the tubular path are located in a medium with a high substrate concentration, which likewise increases the reaction speed. The average productivity of the reactor is therefore altogether dramatically higher than that in stirrer vessel reactors. Simultaneously, i.e. without sacrificing the high productivity, the tubular reactor can be operated such that a complete yield is obtained in the fermenter discharge.

With respect to the process control it proves to be a very great advantage that the distribution of turnover time of the fluid elements is very narrow at turbulent flow (Reynolds number Re ≥ 2500) and at tube diameters of 40 to 120 mm. That is, the turnover time of the fastest and slowest fluid elements is very uniform in contrast to continuously operated stirrer vessels. A turbulent flow can also be realized at lower flow rates, e.g. by installing flow breakers in the tubular path.

The flow developing in the inventive method can be termed a turbulent plug flow. This means that concentration gradients arise with respect to the substrate along the reaction path, i.e. once product is formed it remains substantially within the reactor segment, not increasing the product concentration in the total reactor as in conventional continuous reactors. This avoids premature feedback inhibition.

Furthermore it is possible with the inventive method to add substrate and any further necessary additives in selectively dosed fashion so as to avoid substrate inhibition. This is done for example by feeding only relatively low-concentrated substrate in the beginning of the reaction path and adding further substrate stepwise in dosed fashion along the course of the reaction path so that a maximum concentration is not exceeded but the full fermentation capacity is exploited. The same applies to other additives, for example the lye necessary for pH regulation in the production of lactic acid, such as NaOH or NH₄OH.

The invention also relates to the use of a reactor for carrying out this method having at least one inlet for substrate, microorganism and any further necessary additives and an outlet for product phase, means for retaining or recycling the microorganism and means for controlling temperature, flow and other essential parameters, the reactor being a tubular reactor with an inside diameter that results in a turbulent flow in conjunction with the flow rate of the medium required for the virtually complete reaction of the substrate, and its viscosity, at a Reynolds member of flow of Re ≥ 2500.

The inventive method and the tubular reactor developed for it provide a number of constructional and apparatus-related advantages. The constructional design is extremely simple and inexpensive and also permits easy adaptation to the particular microorganism of interest. Substrate concentration and nutrient-salt concentration, flow rate and cellular substance, pH value and other parameters can be controlled very easily by dosed feeding or removal. The unit can be cleaned and sterilized very reliably since the total inner surface is rinsed with no dead space.

The inventively used tubular reactor has a very high surface-to-volume ratio that permits transfer of heat and a reliable scale-up for every production level. Temperature gradients can build up along the extremely long reaction path so that the temperature-dependent growth and product-forming process can be easily influenced. In connection with the very easily controlled substrate concentration the optimal growth and product-forming conditions can be adjusted in every area of the reactor. Apparatus and measuring devices for process control can be integrated everywhere so the course of the method can be monitored very precisely.

Summing up, the characteristic main feature and the both economically and ecologically novel property of the tubular reactor for fermentative recovery of lactic acid is the possibility of obtaining both a complete yield and high productivity with an altogether very low use of energy.

The fermentation process was optimized with respect to the substrate composition and cell concentrations throuah several experimental intermediate stages in various reactors until very short fermentation periods up to complete reaction in the range of 60 to 90 in were reached. If the reaction times were too long the necessary tube length would be technically uncontrollable. On the basis of these results a tubular reactor with L = 630 m and a tube diameter of 50 mm was ultimately installed during development and provided with the necessary instruments and apparatus for cell retention (membrane, centrifuge) and cell recycling, for process control and for regulation. With a reaction volume of 1.2 m³ and a volume flow rate of fermentation substrate of about 750 l/h the prototype reactor was able to prove experimentally on an almost industrial scale that the concept of fermentative recovery of lactic acid in turbulent tubular flow is suitable for practice and can be realized in terms of production technology. The productivity of the reactor is up to 35 g of lactate/(lh) at a cell density up to 20 g of dry matter/l, whereas the reactors currently used in industry have no more than an average of 1 to 2 g/(lh).

## Claims

1. A method for continuously recovering microbial fermentation products and/or microbial cellular substance, the method being carried out in a suitable medium in a tubular fermentation reactor to which substrate and microorganisms are continuously fed and from which product phase is continuously removed, characterized in that fresh substrate is fed in accordance with a turnover time specific to the organism and that the medium flows turbulently in a plug flow, the flow having a Reynolds number of flow of Re ≥ 2500.

2. The method of claim 1, characterized in that it is used for recovering lactic acid and/or lactic-acid bacteria.

3. The method of claim 1 or 2, characterized in that microorganisms are recycled.

4. The method of any of claims 1 to 3, characterized in that temperature gradients build up along the reaction path for optimal utilization of substrate.

5. The method of any of claims 1 to 4, characterized in that substrate and/or any required additives are added along the reaction path.

6. The method of any of claims 1 to 5, characterized in that the flow rate is adjusted in such a way that the substrate is substantially reacted to product at the end of the tubular reactor.

7. Use of a tubular reactor for continuously recovering microbial fermentation products and/or microbial cellular substance in a method according to claim 1, the reactor comprising at least one inlet for substrate, microorganisms and any further necessary additives and an outlet for product phase, means for recycling the microorganisms and means for monitoring and controlling temperature, flow and other essential parameters, characterized in that the tubular reactor has an inside diameter that results in a turbulent flow in conjunction with the flow rate of the medium required for virtually complete reaction of the substrate, and its viscosity, at a Reynolds number of flow of Re ≥ 2500.

8. The use of claim 7, characterized in that the inside diameter of the reactor tube is 40 to 120 mm.

9. The use of claim 7 or 8 characterized by a total length of the tubular path of 50 to 1000 m.

## Patentansprüche

1. Verfahren zur kontinuierlichen Gewinnung von mikrobischen Fermentationsprodukten und/oder einer mikrobischen zellularen Substanz, welches in einem geeigneten Medium in einem röhrenförmigen Fermentationsreaktor durchgeführt wird, dem Substrat und Mikroorganismen kontinuierlich zugeführt werden und von dem die Produktphase kontinuierlich abgeführt wird, **dadurch gekennzeichnet daß**, frisches Substrat gemäß einer für den Organismus spezifischen Zeit zugeführt wird, und daß das Medium turbulent in einer idealen Strömung fließt, wobei die Strömung einer Reynolds-Zahl der Strömung von Re ≥ 2500 besitzt.

2. Verfahren nach Anspruch 1, **dadurch gekennzeichnet daß** , es zur Gewinnung von Milchsäure und/oder Milchsäurebakterien verwendet wird.

3. Verfahren nach Anspruch 1 oder 2, **dadurch gekennzeichnet daß**, die Mikroorganismen recycelt werden.

4. Verfahren nach einem der Ansprüche 1 bis 3, **dadurch gekennzeichnet, daß** sich Temperaturgradienten längs der Reaktionsbahn für eine optimale Verwendung des Substrats aufbauen.

5. Verfahren nach einem der Ansprüche 1 bis 4, **dadurch gekennzeichnet daß**, Sustrat und/oder irgend erforderliche Additive längs der Reaktionsbahn zugesetzt werden.

6. Verfahren nach einem der Ansprüche 1 bis 5, **dadurch gekennzeichnet, daß** die Strömungsrate derart eingestellt wird, daß das Substrat im wesentlichen zum Produkt am Ende des röhrenförmigen Reaktors umgesetzt ist.

7. Verwendung eines röhrenförmigen Reaktors für die kontinuierliche Gewinnung mikrobischer Fermentationsprodukte und/oder einer mikrobischen zellularen Substanz in einem Verfahren gemäß Anspruch 1, wobei der Reaktor wenigstens einen Einlaß für das Substrat, die Mikroorganismen und weitere erforderliche Additive sowie einen Auslaß für die Produktphase, eine Einrichtung zum Recykeln der Mikroorganismen und eine Einrichtung zur Aufnahme und zur Steuerung der Temperatur, der Strömung und weiterer wesenticher Parameter aufweist, **dadurch gekennzeichnet, daß** der röhrenförmige Reaktor einen Innendurchmesser aufweist, der resultiert in einer turbulenten Strömung in Verbindung mit der Strömungsgeschwindigkeit des für die eigentliche vollständige Reaktion des Substrats erfoderlichen Mediums und seiner Viskosität bei einer Reynolds-Zahl der Strömung von RE ≥ 2500.

8. Verwendung nach Anspruch 7, **dadurch gekennzeichnet, daß** der Innendurchmesser der Reaktorröhre 40-120 mm beträgt.

9. Verwendung nach Anspuch 7 oder 8, **gekennzeichnet durch** eine vollständige Länge der röhrenförmigen Bahn von 50 bis 1000 m.

## Revendications

1. Procédé d'obtention continue de produits de fermentation microbienne et/ou de substance celulaire microbienne, ledit procédé étant réalisé dans un milieu approprié dans un réacteur tubulaire de fermentation, auquel du substrat et des micro-organismes sont alimentés continuellement et duquel la phase de produit est emmenée, **caractérisé en ce que** du substrat frais est alimenté conformément à une période spécifique pour l'organisme et que le médium coule turbulentement dans un écoulement idéal, l'écoulement ayant un nombre de Reynolds d'écoulement de Re ≥ 2500.

2. Procédé selon la revendication 1, **caractérisé en ce qu**'il est appliqué à obtenir d'acide lactique et/ou de bactéries lacto-acides.

3. Procéde selon la revendication 1 ou 2, **caractérisé en ce que** les micro-organismes sont recyclés.

4. Procéde selon une des revendications 1 à 3, **caractérisé en ce que** de gradients de température se constituent suivant la trajectoire réactionnelle pour une application optimale du substrat.

5. Procéde selon une des revendications 1 à 4, **caractérisé en ce que** le substrat et/ou quelques additifs nécessaires sont alimentés au long de la trajectoire réactionnelle.

6. Procéde selon une des revendications 1 ou 2, **caractérisé en ce que** le rapport d'écoulement est ajusté de sorte que le substrat est essentiellement transformé en produit à la fin du réacteur tubulaire.

7. Emploi d'un réacteur tubulaire pour l'obtention continue de produits de fermentation microbienne et/ou de substance celulaire microbienne dans un procédé selon la revendication 1, le réacteur montrant au moins une entrée pour le substrat, les micro-organismes et autres additifs nécessaires et même une sortie pour le phase de produit, un dispositif de recyclage de micro-organismes et un dispositif d'enregistrement et contrôle de température, d'écoulage et d'autres paramètres essentiels, **caractérisé en ce que** le réacteur tubulaire montre un diamètre intérieur résultant dans un écoulement turbulant en rapport de la vitesse d'écoulement du médium nécessaire pour la propre réaction complète du substrat et sa viscosité en cas d'un nombre de Reynolds d'écoulement de Re ≥ 2500.

8. Emploi selon la revendication 7, **caractérisé en ce que** le diamètre intérieur du tube de réacteur est 40 à 120 mm.

9. Emploi selon la revendication 7 ou 8, **caractérisé par** un longueur complet de la trajéctoire tubulaire de 50 à 1000 m.
